# EUROPEAN PATENT APPLICATION

(11) **EP 4 603 116 A1**
(43) Date of publication of application: **20.08.2025**
(21) Application number: 23883077.2
(22) Date of filing: 25.10.2023
(51) Int. Cl.: A61M 5/142, A61M 5/172, G16H 20/17, G16H 50/20, G16H 40/63, A61M 5/145

(54) **DRUG INJECTION DEVICE AND METHOD**

(30) Priority: 25.10.2022 KR 20220138275; 24.10.2023 KR 20230142906
(71) Applicant: Caremedi Co., Ltd., Seoul 07207 (KR); Sogang University Research & Business Development Foundation, Seoul 04107 (KR)
(72) Inventor: SHIN, Woonsup, Seoul 04065 (KR); ZHU, Enhua, Seoul 04109 (KR); SHIN, Yeong Jong, Gimpo-si Gyeonggi-do 10126 (KR)
(74) Representative: Manitz Finsterwald Patent- und Rechtsanwaltspartnerschaft mbB
(86) International application number: PCT/KR2023/016599
(87) International publication number: WO 2024/090972

(57) **Abstract**

A drug injection device according to the present disclosure includes: a drug injector configured to electrochemically drive an electroosmotic pump to suck a drug from a drug storage and discharge the sucked drug to an injection target; and a controller configured to output, to the drug injector, a control signal corresponding to an amount-based injection sequence that enables the electroosmotic pump to be driven in an immediate injection mode, wherein the amount-based injection sequence includes at least one pulse block that defines a voltage pulse or a current pulse to be applied to the electroosmotic pump, each pulse block defines a pair of pulse signals including a forward pulse and a reverse pulse that are applied to the electroosmotic pump to alternately generate negative pressure and positive pressure, and the electroosmotic pump alternately generates negative pressure and positive pressure for each pulse block to suck and discharge the drug.

## Description

### TECHNICAL FIELD

The technical field of the present disclosure relates to a drug injection device and a drug injection method.

### BACKGROUND

Drugs may be injected into the body in various ways, such as oral, subcutaneous, and intravenous administration, depending on the type of drug and the purpose and method of treatment. A drug injector using a drug pump may automatically inject a drug into the body at a desired speed and dose at a required time. Therefore, a drug injector using a drug pump may be used not only in hospitals or in patients' daily living environments, but also in various forms.

An insulin pump, which is commonly called an insulin injector, is a medical device that supplies insulin into the body from the outside at a predetermined time to accurately control the blood sugar level, similar to the pancreas, for a diabetic patient who does not secrete insulin or secretes only a small amount of insulin.

Such an insulin pump is used by insulin-dependent diabetic patients, and may continuously inject a drug for 24 hours in a state of being attached to the patient. In this way, the insulin injector needs to periodically inject a drug into a diabetic patient for a long period of time. Therefore, technological development is actively underway to miniaturize and automate insulin injectors for the convenience of users.

In general, the insulin injector operates in a basal injection mode in which a drug is injected into a user for a predetermined period of time to maintain the user's blood sugar level consistently. The basal injection mode is set based on the user's usual activity level. However, when the user's blood sugar level changes due to meals or snacks, insulin injection cannot be flexibly adjusted.

This may cause a sharp increase in the user's blood sugar level. Therefore, a method for continuously injecting a drug into a patient, stably injecting an accurate amount of the drug depending on the patient's activity level, and controlling drug injection to suppress blockage in a flow path during the drug injection is required.

### DISCLOSURE OF THE INVENTION

### PROBLEMS TO BE SOLVED BY THE INVENTION

In view of the foregoing, the present disclosure is conceived to provide a method of drug injection in a meal injection mode by a drug injection device based on an electroosmotic pump.

The problems to be solved by the present disclosure are not limited to the above-described problems. There may be other problems to be solved by the present disclosure.

### MEANS FOR SOLVING THE PROBLEMS

As a means for solving the above-described problems, a drug injection device according to an embodiment of the present disclosure includes: a drug injector configured to electrochemically drive an electroosmotic pump to suck a drug from a drug storage and discharge the sucked drug to an injection target; and a controller configured to output, to the drug injector, a control signal corresponding to an injection sequence determined according to an injection mode, wherein the controller outputs a control signal corresponding to an amount-based injection sequence for an immediate meal injection mode and outputs a control signal corresponding to a speed-based injection sequence for an extended meal injection mode, and the electroosmotic pump alternately generates negative pressure and positive pressure in response to the control signals to suck and discharge the drug.

A drug injection method according to an embodiment of the present disclosure includes: a process of setting a control signal corresponding to an amount-based injection sequence for an immediate meal injection mode; a process of setting a control signal corresponding to a speed-based injection sequence for an extended meal injection mode; a process of applying the control signals to the electroosmotic pump; and a process of causing the electroosmotic pump to alternately generate negative pressure and positive pressure for each pulse block in response to the control signal to suck and discharge a drug, wherein the electroosmotic pump alternately generates negative pressure and positive pressure in response to the control signals to suck and discharge the drug.

### EFFECTS OF THE INVENTION

According to the means for solving the problems of the present disclosure, it is possible to control a drug injector including an electroosmotic pump to inject a predetermined amount of drug by setting at least one of an amount-based injection sequence and a speed-based injection sequence based on drug injection conditions.

Also, it is possible to inject a predetermined drug into a user without a pause period by applying a meal injection mode in response to changes in blood sugar level caused by meals during a basal injection mode operation. Thus, it is possible to respond to changes in blood sugar level of the user.

### BRIEF DESCRIPTION OF THE DRAWINGS

**FIG.** 1 is a block diagram schematically illustrating a drug injection device according to an embodiment of the present disclosure.
**FIG. 2** is a block diagram schematically illustrating a configuration of a drug injector illustrated in **FIG. 1****.**
**FIG. 3** is a conceptual diagram schematically showing an amount-based injection sequence according to an embodiment of the present disclosure.
**FIG. 4** is a table showing an example of a plurality of pulse blocks according to an embodiment of the present disclosure.
**FIG. 5** is an example diagram illustrating a signal structure for the amount-based injection sequence according to an embodiment of the present disclosure.
**FIG. 6** is an example diagram illustrating a signal structure for a speed-based injection sequence according to an embodiment of the present disclosure.
**FIG. 7** is a table showing an example of the speed-based injection sequence according to an embodiment of the present disclosure.
**FIG. 8** is an example diagram illustrating a signal structure for the speed-based injection sequence illustrated in **FIG. 7****.**
**FIG. 9** is an example diagram illustrating a signal structure in which an injection sequence for a meal injection mode is applied to the speed-based injection sequence for a basal injection mode illustrated in **FIG. 8****.**
**FIG. 10** is a block diagram schematically illustrating a configuration of an electroosmotic pump illustrated in **FIG. 2****.**
**FIG. 11** is a conceptual diagram schematically illustrating a configuration of a driver illustrated in **FIG. 10****.**
**FIG. 12** is an example diagram schematically illustrating an operation of a driver illustrated in **FIG. 11****.**
**FIG. 13** illustrates an example of application of the drug injection device according to an embodiment of the present disclosure.
**FIG. 14** is a block diagram schematically illustrating a configuration of the drug injection device illustrated in **FIG. 13****.**
**FIG. 15** is a flowchart illustrating a drug injection method according to an embodiment of the present disclosure.

### BEST MODE FOR CARRYING OUT THE INVENTION

Hereafter, embodiments will be described in detail with reference to the accompanying drawings. However, it is to be noted that the present disclosure is not limited to the embodiments but can be embodied in various other ways. Also, the accompanying drawings are provided only for a better understanding of the embodiments disclosed in the present disclosure and are not intended to limit technical ideas disclosed in the present disclosure. In the drawings, parts irrelevant to the description are omitted for the simplicity of explanation, and the size, form and shape of each component illustrated in the drawings can be modified in various ways. Like reference numerals denote like parts throughout the whole document.

Suffixes "module" and "unit" used for components disclosed in the following description are merely intended for easy description of the specification, and the suffixes themselves do not give any special meaning or function. Further, in the following description of the present disclosure, a detailed explanation of known related technologies may be omitted to avoid unnecessarily obscuring the subject matter of the present disclosure.

Throughout the whole document, the term "connected to (contacted with or coupled to)" may be used to designate a connection or coupling of one element to another element and includes both an element being "directly connected to (contacted with or coupled to)" another element and an element being "indirectly connected to (contacted with or coupled to)" another element via another element. Further, through the whole document, the term "comprises or includes" and/or "comprising or including" used in the document means that one or more other components, steps, operation and/or existence or addition of elements are not excluded in addition to the described components, steps, operation and/or elements unless context dictates otherwise.

Further, in describing components of the present disclosure, ordinal numbers such as first, second, etc. can be used only to differentiate the components from each other, but do not limit the sequence or relationship of the components. For example, a first component of the present disclosure may also be referred to as a second component and vice versa.

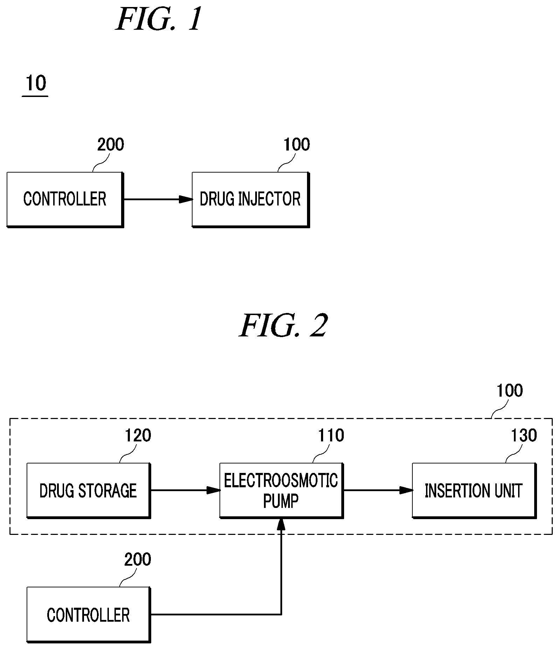

**FIG. 1** is a block diagram schematically illustrating a drug injection device according to an embodiment of the present disclosure, and **FIG. 2** is a block diagram schematically illustrating a configuration of a drug injector illustrated in **FIG. 1****.**

Referring to **FIG. 1** and **FIG. 2****,** a drug injection device 10 according to an embodiment of the present disclosure includes a drug injector 100 and a controller 200.

The drug injector 100 electrochemically drives an electroosmotic pump 110 to suck a drug from a drug storage and discharges the sucked drug to an injection target.

The controller 200 controls a control signal corresponding to an injection sequence determined according to an injection mode and outputs the control signal to the drug injector 100. The controller 200 outputs a control signal corresponding to an amount-based injection sequence for an immediate meal injection mode or outputs a control signal corresponding to a speed-based injection sequence for an extended meal injection mode.

Specifically, when a predetermined event for executing a meal injection mode while the drug injector 100 operates according to a first speed-based injection sequence for a basal injection mode, the controller 200 sets at least one of the amount-based injection sequence and a second speed-based injection sequence for the meal injection mode, temporarily stops outputting a control signal corresponding to the first speed-based injection sequence for the basal injection mode currently being executed, and outputs a control signal corresponding to the amount-based injection sequence and the second speed-based injection sequence for the meal injection mode.

Herein, the basal injection mode is a drug injection mode to continuously inject a drug into the user over a predetermined period of time to maintain the blood sugar level, and the meal injection mode is a drug injection mode to inject a predetermined amount of drug into the user during a meal to maintain the user's blood sugar level within a normal range depending on the meal quantity or type of food.

Meanwhile, the meal injection mode may include the immediate meal injection mode to immediately inject a drug based on the meal quantity or type of food and the extended meal injection mode to continuously inject a drug for a predetermined period of time. The immediate meal injection mode can be applied when the user eats food that immediately affects the blood sugar level, and enables an operation according to the amount-based injection sequence.

In the immediate meal injection mode, when the user sets a drug injection amount based on the user's meal information of the user, an amount-based injection sequence corresponding to the drug injection amount is set. Also, when the user inputs the user's meal information into a bolus calculator, the drug injection amount is calculated according to the type or composition of the meal and a speed-based injection sequence corresponding to the drug injection amount is set.

The extended meal injection mode can be applied when the user continues eating foods over a predetermined period of time, such as at a buffet or multi-course meal, or when the user eats food containing a component that causes a slow change in blood sugar level, and enables an operation according to the speed-based injection sequence.

When the user sets an extended injection period in which the extended meal injection mode continues and an extended injection amount indicating the amount of drug to be injected for the extended injection period based on the user's meal information, it is possible to set a speed-based injection sequence corresponding to the extended injection period and the extended injection amount. Further, when the user's meal information is input into the bolus calculator, the extended injection period and the extended injection amount are calculated according to the type or composition of the meal and the speed-based injection sequence corresponding to the extended injection period and the extended injection amount are set. For example, when the bolus calculator determines that the meal consists primarily of fats and proteins rather than carbohydrates based on the meal information, it may recommend extended injection and calculate the extended injection period and the extended injection amount accordingly.

When the extended injection period and the extended injection amount are determined, the speed-based injection sequence for the extended meal injection mode is set in the same manner as the speed-based injection sequence for the basal injection mode. That is, the speed-based injection sequence is set by setting a drug injection speed to ensure that the extended injection amount of drug is injected for the extended injection period.

The meal injection mode is executed with the immediate meal injection mode by default and composed of one or more of the immediate meal injection mode and the extended meal injection mode depending on the choice of implementation. A detailed configuration of the immediate meal injection mode and the extended meal injection mode will be described again later.

Further, the controller 200 may refer to a data processor which is embedded in hardware and includes a physically structured circuit to perform a function expressed as a code or command included in a program. The data processor embedded in hardware may include, for example, a microprocessor, a central processing unit (CPU), a processor core, a multiprocessor, an application-specific integrated circuit (ASIC), a field programmable gate array (FPGA), a micro control unit (MCU), an embedded processor, and the like, but the scope of the present disclosure is not limited thereto.

Meanwhile, the controller 200 may be embedded solely in the drug injection device 10, or may control the meal injection mode of the drug injection device 10 by being connected to a user device 40, which will be described later, through a communication module and being integrated into the user device 40.

Hereafter, a structure of the amount-based injection sequence and a structure of the speed-based injection sequence will be described.

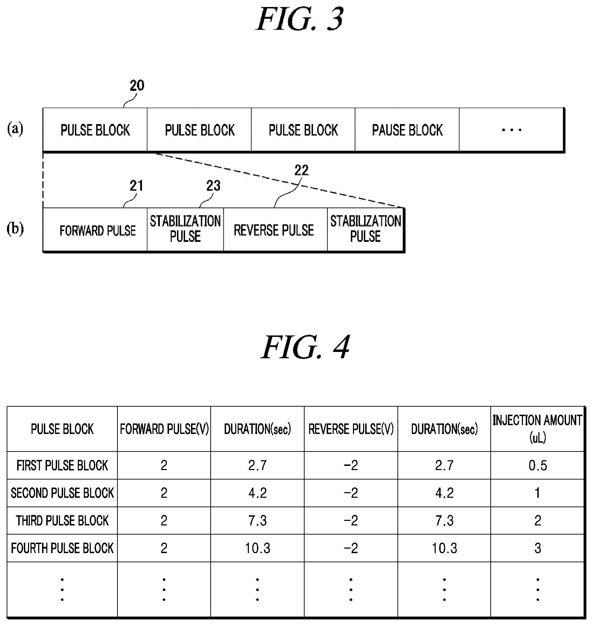

First, a structure of the amount-based injection sequence will be described. **FIG. 3** is a conceptual diagram schematically showing the amount-based injection sequence for the immediate meal injection mode according to an embodiment of the present disclosure. The amount-based injection sequence will be described in detail with reference to **FIG. 3****.**

The amount-based injection sequence is configured by arranging a plurality of pulse blocks 20 that satisfies the drug injection amount based on meal injection conditions or meal information. For example, when the meal information is input into the bolus calculator, the bolus calculator can calculate the drug injection amount based on the meal information and determine the amount-based injection sequence according to the drug injection amount. In this case, the amount-based injection sequence is configured by continuously arranging, without intervals, the plurality of pulse blocks 20 that satisfies the drug injection amount. The injection mode operating according to the amount-based injection sequence may also be referred to as "bolus injection" in conventional insulin injection devices.

In the amount-based injection sequence, the plurality of pulse blocks 20 is continuously arranged without a pause period. Each pulse block 20 defines a voltage pulse or a current pulse to be applied to the electroosmotic pump 110, and the amount of drug to be injected varies depending on its duration.

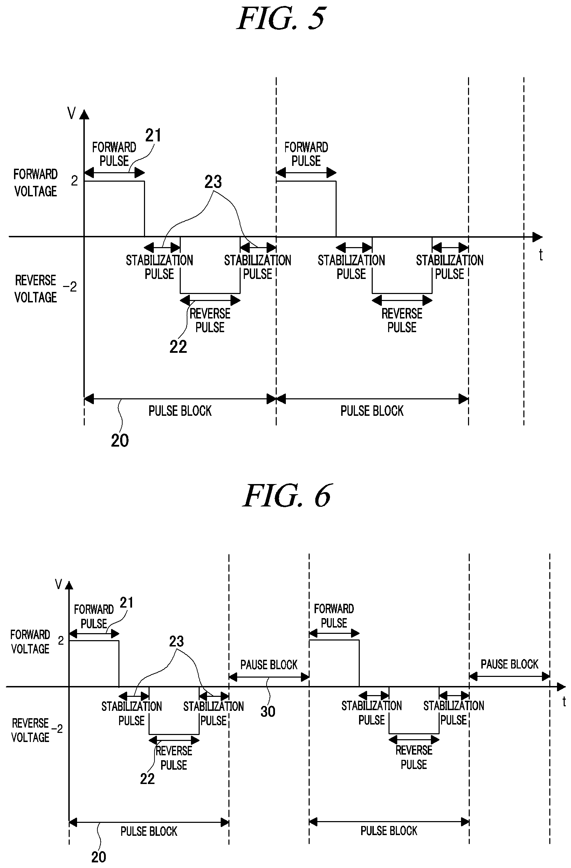

**FIG. 4** is a table showing an example of a plurality of pulse blocks according to an embodiment of the present disclosure, and **FIG. 5** is an example diagram illustrating a signal structure for the amount-based injection sequence according to an embodiment of the present disclosure.

An example of a structure of the amount-based injection sequence will be described with reference to **FIG. 4** and **FIG. 5****.** To satisfy the drug injection conditions including the drug injection amount, the amount-based injection sequence is set as follows. The amount-based injection sequence is configured by continuously arranging M number of pulse blocks 20 (M is a natural number less than or equal to N) selected from among N number of pulse blocks 20 (N is a natural number), which supply different amounts of drug, based on the drug injection amount as shown in **FIG. 4****.**

Herein, the plurality of pulse blocks 20 of the amount-based injection sequence is used in the minimum number required to satisfy the drug injection amount, and among the pulse blocks 20 that satisfy the drug injection amount, a pulse block 20 supplying the largest amount of drug is arranged first. For example, if the drug injection amount is 5 µL, the amount-based injection sequence is composed of a fourth pulse block, which supplies 3 µL, and a third pulse block, which supplies 2 µL. In this case, the fourth pulse block is arranged first, followed by the third pulse block. Also, as soon as the fourth pulse block is completed, the third pulse block is immediately executed.

Hereafter, the pulse block 20 will be described with reference to **FIG. 5****.** Each pulse block 20 includes information about a pair of pulse signals including a forward pulse and a reverse pulse, and includes information about an amplitude of each pulse and a duration for which a pulse is maintained. The pair of pulse signals including the forward pulse and the reverse pulse are applied to the electroosmotic pump 110 to alternately generate negative pressure and positive pressure. Thus, negative pressure and positive pressure are alternately generated for each of the pulse blocks 20 to suck and discharge a drug.

A pair of pulse signals 21 and 22 included in the pulse block 20 may be voltage pulse signals or current pulse signals. A pair of voltage pulse signals may be composed of a forward voltage pulse and a reverse voltage pulse, and a pair of current pulse signals may be composed of a forward current pulse and a reverse current pulse. Herein, the pair of voltage pulse signals may include information about amplitudes and durations of the respective voltage pulses, and the pair of current pulse signals may include information about amplitudes and durations of the respective current pulses. For example, in the amount-based injection sequence illustrated in **FIG. 4B,** the amplitude of a pulse signal may be 2 V, and the duration thereof may be 10 s.

Each pulse block 20 included in the amount-based injection sequence may include voltage pulses having different voltage levels or current pulses having different current levels. Alternatively, each pulse block 20 may include voltage pulses having the same voltage level or current pulses having the same current level, but respective durations may be set differently.

Further, the forward pulse 21 and the reverse pulse 22 included in the pulse block 20 may be set to have the same amplitude and the same duration. Accordingly, the amounts of drug sucked and discharged by the electroosmotic pump 110 may remain the same.

Furthermore, the pair of pulse signals 21 and 22 included in the pulse block 20 may be provided as a constant voltage or a constant current, and the amounts of drug sucked and discharged by each pulse block may be regulated by controlling the duration of a pulse signal provided by a constant voltage or a constant current. For example, the pulse blocks 20 of **FIG. 5** are provided by a constant voltage of 2 V.

Also, the amplitudes and durations of the pair of pulse signals 21 and 22 may be controlled. Accordingly, the amounts of drug sucked and discharged may be regulated to be the same. For example, the amplitude of a forward voltage pulse may be 2 V and the duration thereof may be 10 s, and the amplitude of a subsequent reverse voltage pulse may be set to 1 V and the duration thereof may be set to 20 s. Accordingly, the area of the forward pulse and the area of the reverse pulse are set to be the same to ensure that the amounts of drug sucked and discharged remain the same.

Each pulse block 20 may further include a stabilization pulse 23, and the stabilization pulse 23 is a pulse that maintains a voltage of 0 V for a predetermined period of time after the forward voltage pulse 21 and the reverse voltage pulse 22 are applied. An operation of the electroosmotic pump 110 may be stabilized by the stabilization pulse 23. Herein, when the pulse block 20 of an amount-based injection sequence is composed of a pair of current pulses, the pulse block 20 may further include the stabilization pulse 23 that maintains a current of 0 A for a predetermined period of time after the forward current pulse and the reverse current pulse are applied.

When the control signal corresponding to the amount-based injection sequence is applied to the electroosmotic pump 110, the electroosmotic pump 110 alternately generates negative pressure and positive pressure for each pulse block to suck and discharge the drug. Herein, the control signal may be a signal composed of a pair of voltage pulses including a forward voltage pulse and a reverse voltage pulse in units of pulse blocks 20 or a pair of current pulses including a forward current pulse and a reverse current pulse in units of pulse blocks 20.

Hereafter, a structure of the speed-based injection sequence will be described. FIG. 6 is an example diagram illustrating a signal structure for the speed-based injection sequence for a basal injection mode according to an embodiment of the present disclosure. The speed-based injection sequence is configured by arranging a plurality of pulse blocks that satisfies a drug injection period and a drug injection speed. Herein, the speed-based injection sequence is configured by arranging a plurality of pulse blocks that satisfies a drug injection speed for a unit time (e.g., one hour or 30 minutes) and repeating the plurality of pulse blocks during the drug injection period.

Referring to **FIG. 6****,** the speed-based injection sequence includes at least one pulse block 20 that defines a voltage pulse or a current pulse to be applied to the electroosmotic pump 110 and at least one pause block 30 that maintains a voltage of 0 V or a current of 0 A for a predetermined period of time after the pulse block is applied.

In the basal injection mode, a plurality of speed-based injection sequences is continuously arranged for the set drug injection period. In the basal injection mode, the drug injection period may be divided into a plurality of segments by the user. Herein, each segment includes information about a duration and a drug injection speed for the duration. Each segment is configured by repeatedly arranging the speed-based injection sequence set for the unit time to satisfy the drug injection speed during its duration. The duration and drug injection speed of each segment may be set by the user.

Meanwhile, the speed-based injection sequence satisfying the drug injection period and the drug injection speed is set as follows. First, pulse blocks that satisfy a drug injection amount per unit time need to be arranged to satisfy the drug injection speed. In the present disclosure, various pulse block can be set by using the electroosmotic pump 110. However, during the drug injection period, a plurality of or a predetermined number of pulse blocks need to be arranged whenever possible to minimize a pause period of a pause block arranged after each pulse block and suppress drug drying or blockage. To satisfy these conditions, the speed-based injection sequence may be set as follows.

The speed-based injection sequence is configured by arranging M number of pulse blocks 20 (M is a natural number less than or equal to N) selected from among N number of pulse blocks 20 (N is a natural number greater than 2), which supply different amounts of drug, based on a drug injection speed per unit time. The plurality of pulse blocks 20 and pause blocks 30 of the speed-based injection sequence are arranged such that a duration of each pause block 30 is less than a recommended pause period while satisfying the drug injection speed. Herein, the number of pulse blocks 20 arranged in the speed-based injection sequence may be a predetermined number per unit time or a maximum number that represents the drug injection speed.

Further, the sum of the durations of the plurality of pulse blocks 20 and the plurality of pause blocks 30 following the pulse blocks 20 of the speed-based injection sequence is set to correspond to the unit time. The pulse blocks 20 are arranged such that a pulse block 20 supplying the largest amount of drug is output first, and the arrangement of subsequent pulse blocks 20 may vary depending on the choice of implementation.

Also, the M number of pulse blocks 20 of the speed-based injection sequence may be set to satisfy the drug injection speed while ensuring that the total number of times the selected pulse blocks 20 are used matches a predetermined number or a maximum number and a duration of the pause block 30 following each pulse block 20 is less than the recommended pause period.

Further, in the extended meal injection mode, a predetermined amount of drug is continuously injected for a predetermined period of time. Thus, the speed-based injection sequence is set as an injection sequence for the extended meal injection mode.

Hereafter, an example of an operation of executing the meal injection mode while the basal injection mode is executed will be described.

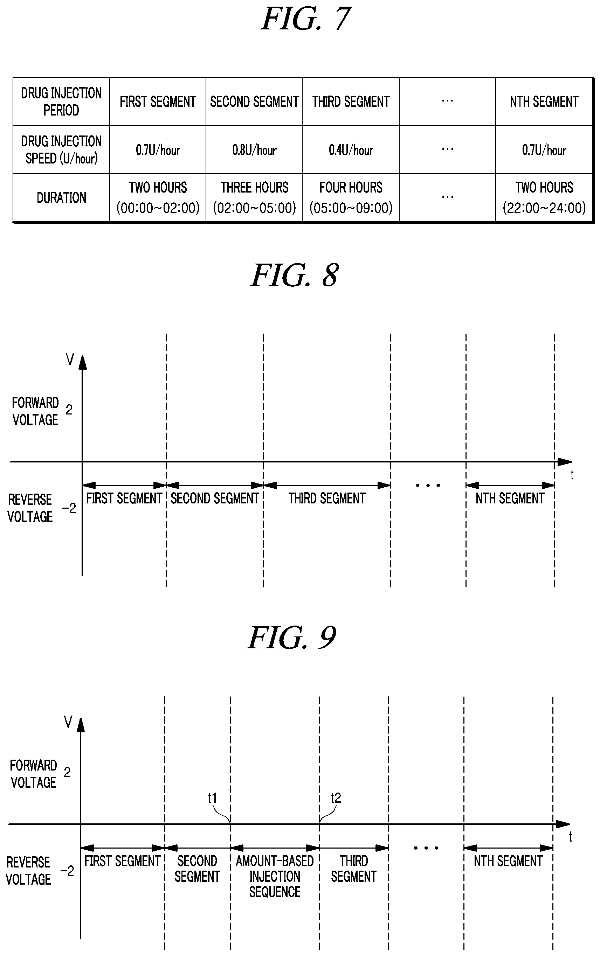

**FIG.** 7 is a table showing an example of the speed-based injection sequence for the basal injection mode according to an embodiment of the present disclosure, and **FIG. 8** is an example diagram illustrating a signal structure for the speed-based injection sequence illustrated in **FIG. 7****.**

An example of the basal injection mode will be described with reference to **FIG. 7** and **FIG.** 8. In the speed-based injection sequence for the basal injection mode shown in **FIG. 7****,** 24 hours are divided into a plurality of segments, each assigned a duration and a drug injection speed. The drug injector 100 performs the basal injection mode by executing the segments in the order shown in **FIG. 8****.**

**FIG. 9** is an example diagram illustrating a signal structure in which an injection sequence for a meal injection mode is applied to the speed-based injection sequence illustrated in **FIG. 8****.** An operation of executing the meal injection mode while the drug injector 100 operates according to the speed-based injection sequence for the basal injection mode will be described with reference to **FIG. 9****.**

First, an operation of executing the immediate meal injection mode while the drug injector 100 operates in the basal injection mode will be described.

As shown in **FIG. 9****,** when the immediate meal injection mode is executed at a time point t1 while the drug injector 100 performs the speed-based injection sequence for the basal injection mode, the controller 200 arranges the amount-based injection sequence for the immediate meal injection mode from the time point t1, stops the speed-based injection sequence of the basal injection mode, and generates a control signal composed of a voltage pulse or a current pulse corresponding to the amount-based injection sequence based on information of the pulse blocks 20 included in the amount-based injection sequence to apply the control signal to the drug injector 100.

Upon receiving the control signal, the drug injector 100 immediately performs the immediate meal injection mode based on the amount-based injection sequence. Then, when the immediate meal injection mode is completed at a time point t2, the controller 200 resumes outputting the control signal for the temporarily stopped speed-based injection sequence at the time point t2 to the drug injector 100.

While the amount-based injection sequence for the immediate meal injection mode is executed, the controller 200 may further perform the omitted operation according to the speed-based injection sequence from the time point t1 to the time point t2 after the time point t2.

Specifically, the controller 200 counts the omitted pulse blocks 20 between the time point t1 and the time point t2 in the speed-based injection sequence and rearranges the omitted pulse blocks 20 in a pause block 30 arranged after the time point t2 to apply the adjusted sequence to the drug injector 100.

The controller 200 rearranges the omitted pulse blocks 20 so as not to exceed a duration of the pause block 30. When the omitted pulse blocks 20 exceed the duration of the pause block 30, some pulse blocks 20 are arranged in the subsequent pause block 30. For example, if three pulse blocks 20 are omitted between the time point t1 and the time point t2 and all the three pulse blocks 20 are arranged in the first pause block 30 after the time point t2, exceeding the duration of the pause block 30, excess pulse blocks 20 are arranged in the subsequent pause block 30.

Also, the controller 200 may omit the operation according to the speed-based injection sequence from the time point t1 to the time point t2 while the amount-based injection sequence for the immediate meal injection mode is executed. At the time point t2 when the operation based on the immediate meal injection mode is completed, the controller 200 resumes outputting the control signal for the speed-based injection sequence.

Hereafter, an operation of executing the extended meal injection mode while the drug injector 100 operates in the basal injection mode will be described.

When the extended meal injection mode is executed while the drug injector 100 operates in the basal injection mode, the user may set a portion of the drug injection amount to be injected according to the amount-based injection sequence for the immediate meal injection mode and the remaining portion to be injected according to the speed-based injection sequence for the extended meal injection mode. The extended meal injection mode is set in response to an additional input of an extended injection period from the user, and a speed-based injection sequence is set accordingly.

In general, when both the immediate meal injection mode and the extended meal injection mode are executed, the amount-based injection sequence is performed first. When the amount-based injection sequence is ended, the speed-based injection sequence is performed. Also, the entire drug injection amount may be injected in the extended meal injection mode.

First, an operation of injecting the entire drug injection amount in the extended meal injection mode will be described with reference to FIG. 9. When the extended meal injection mode is set from the time point t1 to the time point t2 while the drug injector 100 performs the first speed-based injection sequence for the basal injection mode, the controller 200 generates a third speed-based injection sequence, which combines the first speed-based injection sequence arranged between the time point t1 and the time point t2 and the second speed-based injection sequence for the extended meal injection mode, and applies the third speed-based injection sequence to the drug injector 100. Since both the basal injection mode and the extended meal injection mode are set based on speed-based injection sequences, the two sequences can be combined. Therefore, the third speed-based injection sequence generated by combining the first speed-based injection sequence and the second speed-based injection sequence can be arranged between the time point t1 and the time point t2.

For example, referring to **FIG. 7** and **FIG. 9****,** a second segment and a third segment are connected between the time point t1 and the time point t2 in the first speed-based injection sequence, and, thus, portions of the second and third segments are arranged between the time point t1 and the time point t2. A section corresponding to the second segment operates at 0.8 U/h, and a section corresponding to the third segment operates at 0.4 U/h. When the second speed-based injection sequence operates at 1.5 U/h, the controller 200 combines the first and second speed-based injection sequences to generate the third speed-based injection sequence in which the section corresponding to the first segment operates at 2.3 U/h and the section corresponding to the second segment operates at 1.9 U/h.

Alternatively, the controller 200 may not combine the first and second speed-based injection sequences, but may execute only the second speed-based injection sequence and omit an operation according to the first speed-based injection sequence between the time point t1 and the time point t2.

Hereafter, an operation of executing the meal injection mode including both the immediate meal injection mode and the extended meal injection mode during the basal injection mode will be described. When the meal injection mode composed of the immediate meal injection mode and the extended meal injection mode is set between the time point t1 and the time point t2 in the first speed-based injection sequence for the basal injection mode, the amount-based injection sequence for the immediate meal injection mode and the second speed-based injection sequence for the extended meal injection mode are continuously arranged from the time point t1.

Then, the first speed-based injection sequence of the basal injection mode is stopped, and the amount-based injection sequence and the second speed-based injection sequence are executed sequentially. The operation according to the first speed-based injection sequence in a section corresponding to the amount-based injection sequence for the immediate meal injection mode may be rearranged in a pause block 30 arranged after the time point t2 and then executed. Also, the third speed-based injection sequence, which combines the first and second speed-based injection sequences, may be generated in a section corresponding to the second speed-based injection sequence for the extended meal injection mode and executed.

Alternatively, after the amount-based injection sequence and the second speed-based injection sequence are executed sequentially, the controller 200 may omit the operation according to the first speed-based injection sequence between the time point t1 and the time point t2.

In general, the meal injection mode is executed with the immediate meal injection mode by default. When the user selects the extended meal injection mode, the immediate meal injection mode and the extended meal injection mode may be combined and executed.

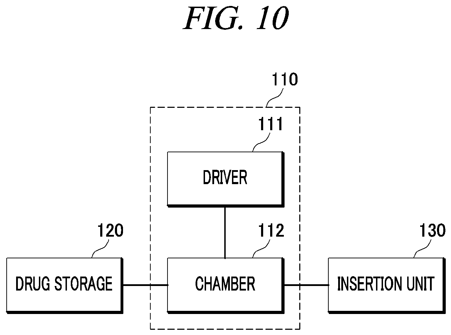

**FIG. 10** is a block diagram schematically illustrating a configuration of an electroosmotic pump illustrated in **FIG. 2****.**

The electroosmotic pump 110 will be described in detail with reference to **FIG. 10****.** The electroosmotic pump 110 includes a driver 111 and a chamber 112. The driver 111 is electrochemically driven in response to a control signal to generate positive pressure and negative pressure and discharges a drug according to the positive pressure and the negative pressure, and the chamber 112 sucks the drug from a drug storage 120 according to the pressure of the driver 111 and then discharges the drug to an insertion unit 130. Herein, the drug needs to be injected into a specific patient and may be, for example, insulin that is injected into a diabetic patient.

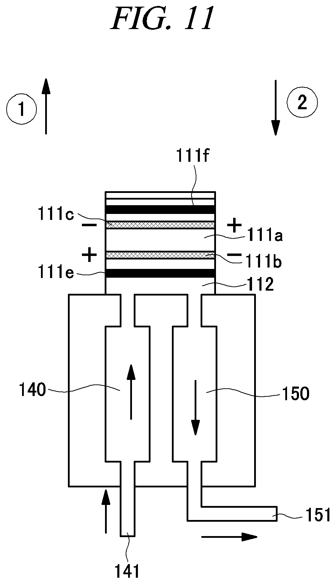

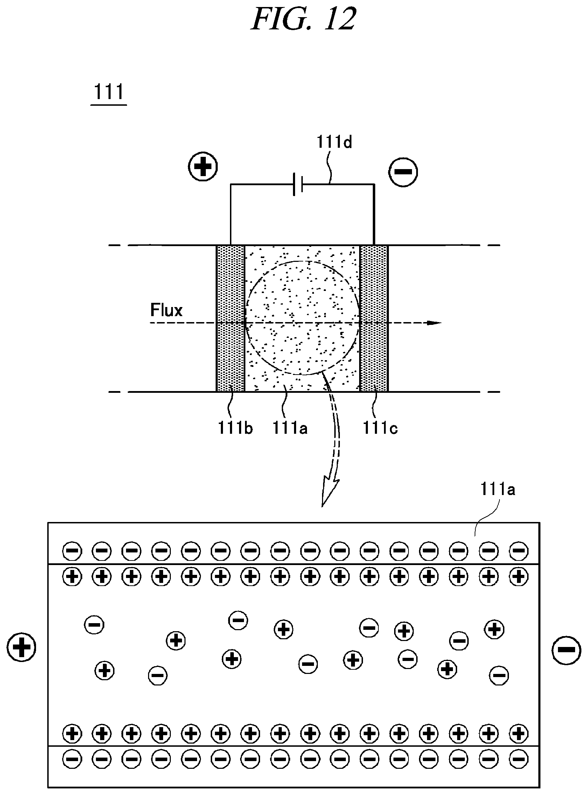

**FIG. 11** is a block diagram schematically illustrating a configuration of a driver illustrated in **FIG. 10****,** and **FIG. 12** is an example diagram schematically illustrating a configuration of the driver illustrated in **FIG. 11****.**

The driver 111 will be described in detail with reference to **FIG. 11** and **FIG. 12****.** The driver 111 is a pump that uses the movement of a fluid according to electroosmosis that occurs when a voltage or current is applied by using electrodes at both ends of a porous film (membrane) 111a. The driver 111 may include the membrane 111a, a power source 111d that applies a voltage or current to a first electrode 111b and a second electrode 111c arranged on both sides of the membrane 111a, and a flow path through which a fluid moves.

Also, the driver 111 may include a first diaphragm 111e arranged adjacent to the first electrode 111b and a second diaphragm 111f arranged adjacent to the second electrode 111c. The first and second diaphragms 111e and 111f are provided respectively one side and the other side of the membrane 111a, and deformed in shape by the movement of a pumping solution as positive pressure and negative pressure are alternately generated. For example, the first diaphragm 111e and the second diaphragm 111f transfer the negative pressure and positive pressure generated by an operation of the membrane 111a to a transfer target fluid. More specifically, when negative pressure is generated, at least a part of the first diaphragm 111e and the second diaphragm 111f moves backwards (i.e., moves in a direction ①) such that the transfer target fluid is sucked to the chamber 112, and when positive pressure is generated, at least a part of the first diaphragm 111e and the second diaphragm 111f moves forwards (i.e., moves in a direction ②) such that the transfer target fluid is discharged from the chamber 112.

Silica, glass, and the like are generally used as a material of the porous film 111a, and when the silica and glass are immersed in an aqueous solution, surfaces thereof are negatively charged. There are many paths, through which fluids may pass, in the porous film 111a, and when one of the paths is enlarged, a surface of the fluid path with negative charges (bound anions) may be in a state in which the negative charges are balanced by mobile cations having positive charges. In this state, when a positive voltage is applied to the first electrode 111b and a negative voltage is applied to the second electrode 111c, negative pressure is generated, and the fluid inside the driver 111 moves in the direction ① due to the negative pressure. In this case, a drug is sucked through an inflow path 141 and flows into the chamber 112 through an inflow valve 140. In this case, an outflow valve 150 is closed such that the negative pressure is not transferred to an outflow path 151. When a negative voltage is applied to the first electrode 111b and a positive voltage is applied to the second electrode 111c, the positive pressure in an opposite direction is generated by a reversible electrochemical reaction. The fluid inside the driver 111 moves in the direction ② by the positive pressure. In this case, the drug stored in the chamber 112 is injected into a target through the outflow valve 150 and the outflow path 151. In this case, the inflow valve 140 is closed such that the positive pressure is not transferred to the inflow path 141.

This phenomenon is called electroosmosis, and a pump that uses this principle is the electroosmotic pump 110.

An electrode used for the driver 111 may be implemented by a platinum (Pt) mesh, such as a porous electrode, porous carbon paper or carbon cloth, or various electrode materials applied onto a porous structure so as to smoothly move a fluid.

Also, the electrode used for the driver 111 may be coated with various fixable materials onto an impermeable substrate material by drop coating or spin coating. In this case, the impermeable substrate material is a plate-shaped substrate including at least one of a conductive material, a semiconductor material, and a non-conductive material, and an electrode material coated thereon may include metal, metal oxide, a conducting polymer, metal hexacyanoferrate, a carbon nanostructure, or a composite thereof.

When polarities of a voltage or current are alternately supplied to the first electrode 111b and the second electrode 111c of the driver 111, reversible electrochemical reactions occur in forward and reverse directions. As the forward and reverse electrochemical reactions occur repeatedly, the fluid inside the electroosmotic pump 110 repeatedly reciprocates back and forth. Further, the electrode materials of the first electrode 111b and the second electrode 111c are repeatedly consumed and regenerated by the repetitive reversible electrochemical reactions in the forward and reverse directions. If the inflow valve 140 and the outflow valve 150 are coupled to the chamber 112 of the electroosmotic pump 110, the drug in the drug storage 120 is sucked through the inflow path 141 and stored in the chamber 112 through the inflow valve 140 during the inflow operation. Furthermore, the drug stored in the chamber 112 is discharged to the insertion unit 130 through the outflow valve 150 and the outflow path 151 during the outflow operation.

**Therefore,** the pressure generated by the electroosmotic pump 110 and the volume of the drug discharged from the electroosmotic pump 110 can be controlled by controlling the amplitudes and application times of voltages or currents applied to the first and second electrodes 111b and 111c.

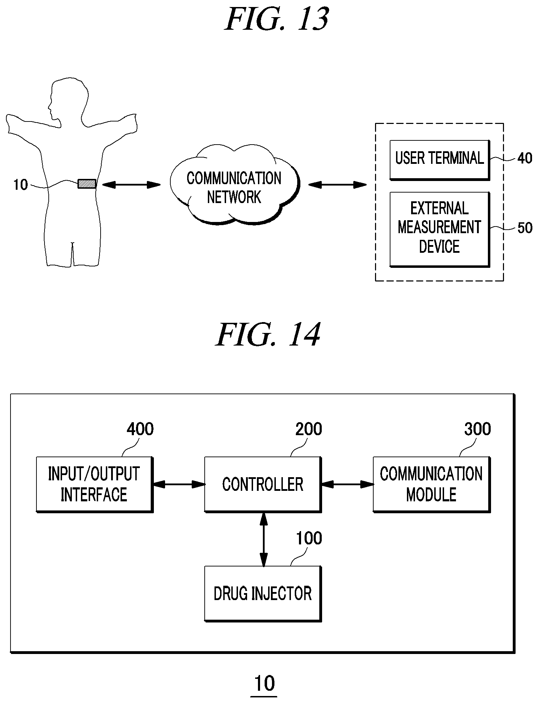

**FIG. 13** illustrates an example of application of the drug injection device according to an embodiment of the present disclosure, and **FIG. 14** is a block diagram schematically illustrating a configuration of the drug injection device illustrated in **FIG. 13****.** An operation of the drug injection device 10 will be described in detail with reference to **FIG. 13** and **FIG. 14****.**

The drug injection device 10 receives predetermined information from the user or an external device and sets at least one of the amount-based injection sequence for the immediate meal injection mode and the speed-based injection sequence for the extended meal injection mode based on the information. Hereafter, the process of setting at least one of the amount-based injection sequence for the immediate meal injection mode and the speed-based injection sequence for the extended meal injection mode will be described.

The drug injection device 10 may detect an event requesting the activation of the meal injection mode and set at least one of the amount-based injection sequence and the speed-based injection sequence accordingly. To detect such an event, the drug injection device 10 may further include a communication module 300 and a user input/output interface 400.

When an event requesting the activation of the meal injection mode is detected via the user device 40 communicatively connected to the communication module 300 or via the user input/output interface 400 executed by the controller 200, the controller 200 allows the user to input a drug injection amount as meal injection conditions or to input meal information to determine the drug injection amount based on the user's meal information through the bolus calculator and thus set at least one of the amount-based injection sequence and the speed-based injection sequence. Herein, the meal information may include information such as the type and quantity of food to be consumed by the user.

The controller 200 receives a selection for at least one of the immediate meal injection mode and the extended meal injection mode through the user input/output interface 400, and sets at least one of the amount-based injection sequence and the speed-based injection sequence based on the selection. When only the immediate meal injection mode is selected through the user interface 400, the controller 200 sets the amount-based injection sequence corresponding to drug injection amount.

When the extended meal injection mode is selected through the user interface 400, the controller 200 may set a portion of the drug injection amount to be injected according to the amount-based injection sequence for the immediate meal injection mode and the remaining portion to be injected according to the speed-based injection sequence for the extended meal injection mode according to the user setting. In this case, the controller 200 may set the speed-based injection sequence in response to an additional input of an extended injection period through the user interface 400. In general, when both the immediate meal injection mode and the extended meal injection mode are executed, the amount-based injection sequence is performed first. When the amount-based injection sequence is ended, the speed-based injection sequence is performed.

Further, the meal injection mode is executed with the immediate meal injection mode by default. Thus, if the extended meal injection mode is not selected, only the immediate meal injection mode is executed.

Furthermore, at least one of the amount-based injection sequence and the speed-based injection sequence may not be set based on the meal injection conditions or meal information, but may be directly selected by the user through the user device 40 or may be calculated by an external computing device and received based on the user's past use history of the drug injection device.

The user device 40 connected to the drug injection device 10 may be implemented by a computer or portable device that can be connected to the drug injection device 10 through wireless communication. Herein, the computer includes, for example, a desktop computer, a laptop computer, and the like equipped with a web browser, and the portable device is, for example, a wireless communication device that ensures portability and mobility and may include all kinds of handheld wireless communication devices, such as various smartphones, tablet personal computers (PCs), and smart watches. The user device 40 is managed by a wearer of the drug injection device 10 or a medical professional, and drug injection conditions or meal information may be set through an application executed on the user device 40.

The user input/output interface module 400 may include a physical input/output button coupled to an external housing including the drug injection device 10, and a signal processing circuit that transmits a signal generated according to an operation of the physical input/output button to the controller 200. Alternatively, the user input/output interface module 400 may output a setting user interface (UI) that guides the user to input information about the meal injection conditions or meal information through a display in the form of a touch screen.

Then, when an operation based on the meal injection mode is completed, the controller 200 resumes outputting a control signal corresponding to the speed-based injection sequence for the basal injection mode, omits an operation according to the speed-based injection sequence which is scheduled to be performed during the pause period, and performs an operation according to the speed-based injection sequence for the basal injection mode which is scheduled to be performed immediately after the operation according to the injection sequence for the meal injection mode is completed.

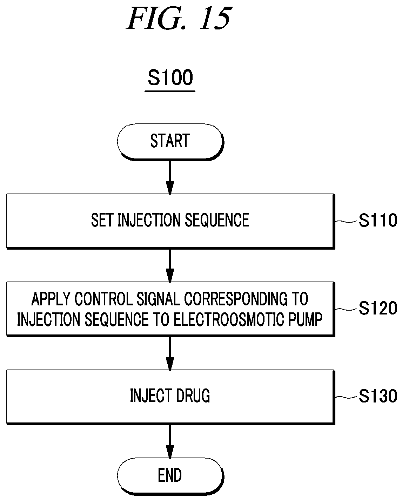

**FIG. 15** is a flowchart illustrating a drug injection method according to an embodiment of the present disclosure.

Referring to **FIG. 1, FIG. 2** and **FIG. 15****,** in a drug injection method S100 according to an embodiment of the present disclosure, the controller 200 sets a control signal corresponding to the amount-based injection sequence for the immediate meal injection mode or sets a control signal corresponding to the speed-based injection sequence for the extended meal injection mode (process S110), and applies a control signal corresponding to the injection sequence to the electroosmotic pump 110 (process S120). Thereafter, in response to the control signal, the electroosmotic pump 110 alternately generates negative pressure and positive pressure for each pulse block to suck and discharge a drug and inject the drug into a user (process S130).

Hereafter, each process will be described in detail.

In the process of setting the control signal (process S110), the drug injection device 10, which is operating based on the speed-based injection sequence for the basal injection mode, receives predetermined information from the user or an external device and sets at least one of the amount-based injection sequence and the speed-based injection sequence for the meal injection mode based on the information.

Specifically, the process of setting the control signal (process S110) will be described. When the drug injection device 10 detects an event requesting the activation of the meal injection mode via the user device 40 communicatively connected to the communication module 300 or via the user input/output interface 400 executed by the controller 200, the controller 200 allows the user to input a drug injection amount as meal injection conditions or to input meal information via the user device 40 or the user input/output interface 400.

Further, the controller 200 may set at least one of the amount-based injection sequence for the immediate meal injection mode and the speed-based injection sequence for the extended meal injection mode based on the meal injection conditions input by the user. Alternatively, based on the meal injection conditions input by the user, the controller 200 sets the amount-based injection sequence according to the drug injection amount calculated by the bolus calculator or sets the speed-based injection sequence according to the drug injection period and the drug injection amount calculated by the bolus calculator. Herein, the meal information may include information such as the type and quantity of food to be consumed by the user.

Furthermore, in the process of setting at least one of the amount-based injection sequence for the immediate meal injection mode and the speed-based injection sequence for the extended meal injection mode, the controller 200 receives a selection for at least one of the immediate meal injection mode and the extended meal injection mode through the user input/output interface 400, and sets at least one of the amount-based injection sequence and the speed-based injection sequence based on the selection.

Herein, the meal injection mode is executed with the immediate meal injection mode by default. Thus, if the extended meal injection mode is not selected, the controller 200 sets the amount-based injection sequence for the drug injection amount and executes the immediate meal injection mode.

Also, the controller 200 may set the amount-based injection sequence or the speed-based injection sequence with reference to a table that stores a plurality of amount-based injection sequences or speed-based injection sequences based on the meal injection conditions or meal information, or the amount-based injection sequence or the speed-based injection sequence may be directly selected by the user through the user device 40 or may be calculated by an external computing device and received based on the user's past use history of the drug injection device instead of the meal injection conditions or meal information.

In the process of applying the control signal to the electroosmotic pump (process S120), when the drug injector 100 operates in the basal injection mode according to the speed-based injection sequence, the controller 200 temporarily stops outputting the control signal corresponding to the speed-based injection sequence and outputs the control signal corresponding to the injection sequence for the meal injection mode to the drug injector 100.

Further, in the process of injecting the drug into the user (process S130), the control signal corresponding to the amount-based injection sequence may be composed of a pair of voltage pulses including a forward voltage pulse and a reverse voltage pulse in units of pulse blocks or a pair of current pulses including a forward current pulse and a reverse current pulse in units of pulse blocks. In response to the control signal, the electroosmotic pump may alternately generate negative pressure and positive pressure for each pulse block to suck and discharge a drug and inject the drug into the user.

Then, when the meal injection mode is ended, the controller 200 resumes outputting the control signal corresponding to the speed-based injection sequence for the basal injection mode that was temporarily stopped, and may further perform or omit the operation according to the speed-based injection sequence for the basal injection mode which is scheduled to be performed during the pause period.

The operation according to the speed-based injection sequence for the basal injection mode which is scheduled to be performed during the pause period of the meal injection mode may vary depending on operations performed in the meal injection mode.

First, a case where the immediate meal injection mode is executed during the operation based on the basal injection mode will be described with reference to FIG. 9. When the amount-based injection sequence for the immediate meal injection mode is set between the time point t1 and the time point t2 in the speed-based injection sequence for the basal injection mode, an operation according to the speed-based injection sequence which is scheduled to be performed between the time point t1 and the time point t2 may be rearranged after the time point t2 and then executed.

Hereafter, a case where the extended meal injection mode is executed during the operation based on the basal injection mode will be described. When the second speed-based injection sequence for the extended meal injection mode is set between the time point t1 and the time point t2 in the first speed-based injection sequence for the basal injection mode, the third speed-based injection sequence, which combines the first speed-based injection sequence for the basal injection mode and the second speed-based injection sequence for the extended meal injection mode, may be generated and then executed accordingly.

Hereafter, a case where both the immediate meal injection mode and the extended meal injection mode are executed during the operation based on the basal injection mode will be described. When both the amount-based injection sequence for the immediate meal injection mode and the second speed-based injection sequence for the extended meal injection mode are set between the time point t1 and the time point t2 in the first speed-based injection sequence for the basal injection mode, the operation according to the first speed-based injection sequence in a section corresponding to the amount-based injection sequence may be rearranged after the time point t2 and then executed and the third speed-based injection sequence, which combines the first speed-based injection sequence in a section corresponding to the second speed-based injection sequence with the second speed-based injection sequence, may be generated and then executed accordingly.

Furthermore, an operation, which is set to be performed in the meal injection mode, may be omitted between the time point t1 to the time point t2 in the basal injection mode and may be performed from the time point t2.

The method according to an embodiment of the present disclosure can be embodied in a storage medium including instruction codes executable by a computer such as a program module executed by the computer. A computer-readable medium can be any usable medium which can be accessed by the computer and includes all volatile/non-volatile and removable/non-removable media. Further, the computer-readable medium may include all computer storage media. The computer storage media include all volatile/non-volatile and removable/non-removable media embodied by a certain method or technology for storing information such as computer-readable instruction code, a data structure, a program module or other data.

The method and system of the present disclosure have been explained in relation to a specific embodiment, but their components or a part or all of their operations can be embodied by using a computer system having general-purpose hardware architecture.

It would be understood by a person with ordinary skill in the art that various changes and modifications may be made based on the above description without changing technical conception and essential features of the present disclosure. Thus, it is clear that the above-described embodiments are illustrative in all aspects and do not limit the present disclosure. The scope of the present disclosure is defined by the following claims. It shall be understood that all modifications and embodiments conceived from the meaning and scope of the claims and their equivalents are included in the scope of the present disclosure.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. A drug injection device, comprising:
a drug injector configured to electrochemically drive an electroosmotic pump to suck a drug from a drug storage and discharge the sucked drug to an injection target; and
a controller configured to output, to the drug injector, a control signal corresponding to an injection sequence determined according to an injection mode,
wherein the controller outputs a control signal corresponding to an amount-based injection sequence for an immediate meal injection mode or outputs a control signal corresponding to a speed-based injection sequence for an extended meal injection mode, and
the electroosmotic pump alternately generates negative pressure and positive pressure in response to the control signals to suck and discharge the drug.

2. The drug injection device of Claim 1,
wherein when a predetermined event occurs, the controller stops an operation based on a first speed-based injection sequence for a basal injection mode and outputs a control signal corresponding to a second speed-based injection sequence for the extended meal injection mode or the amount-based injection sequence for the immediate meal injection mode.

3. The drug injection device of Claim 2,
wherein when an operation based on the immediate meal injection mode is completed, the controller resumes outputting the control signal for the first speed-based injection sequence that was temporarily stopped, and
the controller performs an operation according to the first speed-based injection sequence which is scheduled to be performed during the pause period immediately after the operation based on the immediate meal injection mode is completed.

4. The drug injection device of Claim 2,
wherein the controller executes a third speed-based injection sequence, which combines the first speed-based injection sequence and the second speed-based injection sequence, during an operation based on the extended meal injection mode.

5. The drug injection device of Claim 2,
wherein when an operation based on the extended meal injection mode or the immediate meal injection mode is completed, the controller resumes outputting the control signal for the first speed-based injection sequence that was temporarily stopped, and
the controller omits an operation according to the first speed-based injection sequence which is scheduled to be performed during the pause period and performs an operation according to the first speed-based injection sequence which is scheduled to be performed immediately after the operation based on the extended meal injection mode or the immediate meal injection mode is completed.

6. The drug injection device of Claim 1,
wherein the speed-based injection sequence includes at least one pulse block that defines a voltage pulse or a current pulse to be applied to the electroosmotic pump and at least one pause block that maintains a voltage of 0 V or a current of 0 A for a predetermined period of time after the pulse block is applied,
each pulse block defines a pair of pulse signals including a forward pulse and a reverse pulse that are applied to the electroosmotic pump to alternately generate negative pressure and positive pressure, and
the electroosmotic pump alternately generates negative pressure and positive pressure for each pulse block to suck and discharge the drug.

7. The drug injection device of Claim 6,
wherein the speed-based injection sequence is configured by arranging a plurality of pulse blocks that satisfies a drug injection speed based on drug injection conditions including a drug injection period and the drug injection speed, and
the sum of durations of the plurality of pulse blocks and a plurality of pause blocks following the pulse blocks is set to correspond to the drug injection period.

8. The drug injection device of Claim 6,
wherein the speed-based injection sequence is configured by arranging a plurality of pulse blocks and pause blocks for a unit time, and
the controller repeatedly supplies the speed-based injection sequence during the drug injection period.

9. The drug injection device of Claim 7,
wherein a duration of each pause block in the speed-based injection sequence is less than a recommended pause period.

10. The drug injection device of Claim 7,
wherein in the speed-based injection sequence,
a pulse block supplying the largest amount of drug among a plurality of pulse blocks is output first.

11. The drug injection device of Claim 6,
wherein the speed-based injection sequence is configured by arranging M number of pulse blocks (M is a natural number less than or equal to N) selected from among N number of pulse blocks (N is a natural number), which supply different amounts of drug, based on drug injection conditions,
the drug injection conditions include a drug injection period and a drug injection speed, and
the sum of durations of the M number of selected pulse blocks and a plurality of pause blocks following the pulse blocks is set to correspond to the drug injection period.

12. The drug injection device of Claim 6,
wherein the M number of pulse blocks of the speed-based injection sequence are set to satisfy the drug injection speed of drug injection conditions while ensuring that the total number of times the selected pulse blocks are used matches a predetermined number or a maximum number and a duration of the pause block following each pulse block is less than a recommended pause period.

13. The drug injection device of Claim 7,
wherein the speed-based injection sequence is configured by arranging a pulse block and a pause block assigned to each of a plurality of segments into which the drug injection period is divided in predetermined time units in order for the drug injection speed to be the same for some segments and to be different for some segments.

14. The drug injection device of Claim 6,
wherein the one pulse block and the one pause block form a group and a duration of the group is set to a first time, and
a duration of the pause block is set to a second time determined by subtracting a duration of the pulse block from the first time.

15. The drug injection device of Claim 1,
wherein the amount-based injection sequence includes at least one pulse block that defines a voltage pulse or a current pulse to be applied to the electroosmotic pump,
each pulse block defines a pair of pulse signals including a forward pulse and a reverse pulse that are applied to the electroosmotic pump to alternately generate negative pressure and positive pressure, and
the electroosmotic pump alternately generates negative pressure and positive pressure for each pulse block to suck and discharge the drug.

16. The drug injection device of Claim 15,
wherein the amount-based injection sequence is configured by arranging M number of pulse blocks (M is a natural number less than or equal to N) selected from among N number of pulse blocks (N is a natural number), which supply different amounts of drug, and
a maximum number of pulse blocks that satisfy a target drug injection amount are arranged first.

17. The drug injection device of Claim 15,
wherein in the amount-based injection sequence,
a maximum number of pulse blocks that satisfy a target drug injection amount are arranged first, and
if the amount-based injection sequence includes a plurality of pulse blocks, the pulse blocks are continuously arranged.

18. The drug injection device of Claim 15,
wherein the pair of pulse signals are composed of a pair of voltage pulses including a forward voltage pulse and a reverse voltage pulse, and include a stabilization pulse that maintains a voltage of 0 V for a predetermined period of time after the forward voltage pulse and the reverse voltage pulse are applied, or
the pair of pulse signals are composed of a pair of current pulses including a forward current pulse and a reverse current pulse, and include a stabilization pulse that maintains a current of 0 A for a predetermined period of time after the forward current pulse and the reverse current pulse are applied.

19. The drug injection device of Claim 1,
wherein when an event requesting activation of a meal injection mode is detected via a user interface executed by the controller, the controller allows a user to input a drug injection amount as meal injection conditions via the user interface or to determine a drug injection amount based on the user's meal information through a bolus calculator, and
the controller sets the amount-based injection sequence or the speed-based injection sequence that satisfies the drug injection amount.

20. The drug injection device of Claim 19,
wherein the controller receives an input of at least one of an immediate injection amount and an extended injection period through the user interface, and
the controller generates at least one of an amount-based injection sequence based on the immediate injection amount and a speed-based injection sequence for a drug injection speed based on the remaining injection amount except the immediate injection amount from the drug injection amount and the extended injection period, and outputs the generated sequence as the control signal.

21. The drug injection device of Claim 1, further comprising:
a driver that is electrochemically driven in response to the control signal to alternately generate positive pressure and negative pressure; and
a chamber that sucks the drug from the drug storage according to a pressure of the driver and then discharges the drug to an insertion unit.

22. The drug injection device of Claim 1,
wherein the controller sets the amount-based injection sequence or the speed-based injection sequence with reference to at least one of a table that stores a plurality of amount-based injection sequences for each drug injection amount and a table that stores a plurality of speed-based injection sequences for each drug injection speed.

23. A drug injection method performed by a drug injection device including an electroosmotic pump, comprising:
setting a control signal corresponding to an amount-based injection sequence for an immediate meal injection mode and setting a control signal corresponding to a speed-based injection sequence for an extended meal injection mode;
applying the control signal to the electroosmotic pump; and
causing the electroosmotic pump to alternately generate negative pressure and positive pressure for each pulse block in response to the control signal to suck and discharge a drug,
wherein the electroosmotic pump alternately generates negative pressure and positive pressure in response to the control signals to suck and discharge the drug.

24. The drug injection method of Claim 23,
wherein in the process of applying the control signal to the electroosmotic pump,
when a predetermined event occurs, an operation based on a first speed-based injection sequence for a basal injection mode is stopped, and
a control signal corresponding to a second speed-based injection sequence for the extended meal injection mode or the amount-based injection sequence for the immediate meal injection mode is output.

25. The drug injection method of Claim 24,
wherein in the process of applying the control signal to the electroosmotic pump,
when an operation based on the immediate meal injection mode is completed, outputting the control signal for the first speed-based injection sequence that was temporarily stopped is resumed, and
an operation according to the first speed-based injection sequence which is scheduled to be performed during the pause period immediately after the operation based on the immediate meal injection mode is completed is performed.

26. The drug injection method of Claim 24,
wherein in the process of applying the control signal to the electroosmotic pump,
a third speed-based injection sequence, which combines the first speed-based injection sequence and the second speed-based injection sequence, is executed during an operation based on the extended meal injection mode.

27. The drug injection method of Claim 24,
wherein in the process of applying the control signal to the electroosmotic pump,
when an operation based on the extended meal injection mode or the immediate meal injection mode is completed, outputting the control signal for the first speed-based injection sequence that was temporarily stopped is resumed, and
an operation according to the first speed-based injection sequence which is scheduled to be performed during the pause period is omitted and an operation according to the first speed-based injection sequence which is scheduled to be performed immediately after the operation based on the extended meal injection mode or the immediate meal injection mode is completed is performed.

28. The drug injection method of Claim 23,
wherein in the process of setting the control signal,
when an event requesting activation of a meal injection mode is detected via a user interface, a user is allowed to input a drug injection amount as meal injection conditions via the user interface or to determine a drug injection amount based on the user's meal information through a bolus calculator, and
the control signal corresponding to the amount-based injection sequence or the speed-based injection sequence that satisfies the drug injection amount is set.

29. The drug injection method of Claim 27,
wherein in the process of setting the control signal,
at least one of an immediate injection amount and an extended injection period is input through the user interface, and
at least one of an amount-based injection sequence based on the immediate injection amount and a speed-based injection sequence for a drug injection speed based on the remaining injection amount except the immediate injection amount from the drug injection amount and the extended injection period is generated and set as the control signal.
